(19) Europäisches Patentamt
European Patent Office
Office européen des brevets

(11) **EP 2 382 968 A1**

(12) **EUROPEAN PATENT APPLICATION**

(43) Date of publication:
**02.11.2011 Bulletin 2011/44**

(51) Int Cl.:
*A61K 9/16* $^{(2006.01)}$    *A61K 31/575* $^{(2006.01)}$

(21) Application number: **10157866.4**

(22) Date of filing: **26.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicant: **Abdi Ibrahim Ilac Sanayi ve Ticaret Anonim Sirketi**
**Istanbul (TR)**

(72) Inventors:
 • **Farshi, Farhad**
  **34555 ISTANBUL (TR)**

 • **Avci, Recep**
  **34555 ISTANBUL (TR)**
 • **Apari, Serdar**
  **34555 ISTANBUL (TR)**
 • **Koc, Fikret**
  **34555 ISTANBUL (TR)**
 • **Soylemez, Serdar**
  **34555 ISTANBUL (TR)**
 • **Baris, Sehriban**
  **Kucukcekmece, ISTANBUL (TR)**

(54) **Particle size distribution of fusidic acid granules**

(57)    The present invention relates to a tablet of fusidic acid or a salt thereof characterized in that said tablet s prepared form a mass of granules which have specific particle and granule size distribution.

EP 2 382 968 A1

## Description

### Technical Field

The present invention relates to a tablet of fusidic acid or a salt thereof and a pharmaceutically acceptable excipient or mixtures of excipients.

### Background Art

Fusidic acid, chemically (3α, 4α, 8α, 9α, 11α, 13α, 14α, 16α, 17Z)-16-(Acetyloxy)-3,11-dihydroxy-29-nordammara-17(20), 24-dien-21-oic acid, is an antibacterial agent. It is a well-known antibiotic with a unique steroid-like tetracyclic ring system structure, and it is the most potent of a small family of steroidal antibiotics, the fusidanes. It is produced by fermentation under controlled conditions of the fungus Fusidium Coccineum.

The excellent distribution in various tissues, low degree of toxicity and allergic reactions and the absence cross-resistance with other clinically used antibiotics has made fusidic acid a highly valuable antibiotic, especially for skin and eye infections. The drug is used clinically both in its acid form, and as the sodium salt (Fusidin®), however Fusidin® is more favored one because of its better solubility in water, enabling a fast absorption from gastro-intestinal tract. As a result, it is more preferable to use sodium salt of fusidin in oral solid forms.

Fusidin® has the actions and uses of fusidic acid, and it has been shown that it ameliorates the course of several organ-specific immuno-inflammatory diseases such as chronic uveitis, Behcet's disease, type I diabetes mellitus, Guillain-Barre syndrome, hepatitis, sepsis, pancreatitis, formalin-induced edema, multiple sclerosis, and scleroderma, whereby fucidin formulations have a great importance in pharmaceutical production.

Fusidin® can be presented in various formulations that differ significantly in their pharmacokinetic behaviors such as oral tablets, oral suspensions, intravenous formulations and topical preparation. Considering oral tablets, many of the early clinical studies were performed with capsule containing sodium fusidate. This was also the formulation marketed for many years in several countries. It is currently available as an oral tablet containing the sodium salt. Originally the sodium salt was available as an enteric-coated form but later it was reformulated as a film-coated tablet that appears to be better tolerated and gives higher blood levels.

Fusidic acid sodium salt was used in capsules as well as in tablets which were coated enterically. However by this enteric coating, the active fusidic acid sodium salt was not released before the tablets reached the part of the gastrointestinal tract in which the enteric coating would be dissolved. Depending on the time of passage through the stomach together with the food and the pH in the gastrointestinal tract, this led to unpredictable variations in the blood concentration of the patient undergoing treatment. Because of these adverse differences in blood concentration, the tablets without enteric coating were produced. Now, sodium fusidate is available in tablet, oral solution and injection form.

PCT/WO9603128 A (LEO PHARMACEUTICALS PRODUCTS LTD. ET.AL.) describes the preparation of fusidic acid sodium salt tablets without an enteric coating by using dry granulation method in which a roller compactor was used. The compacted material so produced was size reduced to form a granulate having a bulk density in the range 0.45 to 0.9 g/m$^3$ which was then formed into tablets.

### Summary of invention

This invention provides eligible particle size distribution and dissolution profiles of a pharmaceutical composition of 500 mg fusidic acid or a salt thereof when compared with pharmaceutical compositions of 2x250 mg fusidic acid or a salt thereof.

### Technical Problem

Adjusting of particle size distribution of sodium fusidate granules for 500 mg pharmaceutical composition especially in tablet form is vital to reach desired in vitro dissolution profiles when compared with 2x250 mg pharmaceutical composition especially in tablet form of sodium fusidate. Without adjusting of particle size of granules, as it is defined in this invention, dissolution profiles of 500 mg of sodium fusidate pharmaceutical composition will not be suitable.

### Solution to Problem

Adjusting of elegant in vitro dissolution profiles is carried out by a specific particle size distribution of sodium fusidate granules.

Distribution of sodium fusidate granules that are retained by separation with different sieves showing retain of 10-35% of granules with a sieve of 0.850 mm, 0-25 % of granules with a sieve of 0.500 mm, 5-30 % of granules with a sieve of 0.180 mm and 10-50 % of granules with a sieve of 0.106 mm

More preferably distribution of sodium fusidate granules that are retained by separation with different sieves showing retain of 20-28% of granules with a sieve of 0.850 mm, 10-18 % of granules with a sieve of 0.500 mm, 13-20 % of granules with a sieve of 0.180 mm and 17-30 % of granules with a sieve of 0.106 mm.

Most preferably distribution of sodium fusidate granules that are retained by separation with different sieves showing retain of 25 % of granules with a sieve of 0.850 mm, 14 % of granules with a sieve of 0.500 mm, 15 % of granules with a sieve of 0.180 mm and 20 % of granules with a sieve of 0.106 mm.

In another aspect, this invention provides an oral dosage form containing fusidic acid or salts thereof , wherein oral dosage form is prepared by using of granules of fusidic acid or salts thereof characterized in that the mass of granules comprising - at least 50% of fusidic acid or a salt thereof granules have diameters greater than 100 $\mu$m and - maximum 50% of fusidic acid or a salt thereof granules have diameters smaller than 100 $\mu$m

Preferably granules of fusidic acid or salts thereof characterized in that the mass of granules comprising - 70% of fusidic acid or a salt thereof granules have diameters greater than 100 $\mu$m and - 30% of fusidic acid or a salt thereof granules have diameters smaller than 100 $\mu$m

## Description of embodiments

The object of the present invention is to provide an improved sodium fusidate tablet with a pharmaceutically acceptable excipient or mixture of excipients, having proper dissolution profile.

In one aspect, the invention provides a tablet comprising sodium fusidate obtainable by different tabletting methods include the as wet-granulation, dry-granulation, and direct compression methods.

In further aspect, the invention provides a sodium fusidate tablet produced by dry granulation process using a special processing equipment known as a roller compactor, whereby an homogenous powder comprising sodium fusidate and other pharmaceutically acceptable excipient or combination of excipients, is produced followed by reducing of the compressed material to the proper size for tablet granulation purposes.

In another aspect, this invention provides an oral dosage form containing fusidic acid or salts thereof , wherein oral dosage form is prepared by using of granules of fusidic acid or salts thereof characterized in that the mass of granules comprising - at least 50% of fusidic acid or a salt thereof granules have diameters greater than 100 $\mu$m and - maximum 50% of fusidic acid or a salt thereof granules have diameters smaller than 100 $\mu$m

Preferably granules of fusidic acid or salts thereof characterized in that the mass of granules comprising - 70% of fusidic acid or a salt thereof granules have diameters greater than 100 $\mu$m and - 30% of fusidic acid or a salt thereof granules have diameters smaller than 100 $\mu$m

In another aspect, this invention provides an oral dosage form containing fusidic acid or salts thereof wherein said dosage form is prepared from granules wherein the mass of granules of fusidic acid or salts thereof characterized in that the mass of granules have following particle size distribution : granules are retained by separation with different sieves showing retain of 10-35% of granules with a sieve of 0.850 mm, 0-25 % of granules with a sieve of 0.500 mm, 5-30 % of granules with a sieve of 0.180 mm and 10-50 % of granules with a sieve of 0.106 mm

More preferably the mass of granules of fusidic acid or salts thereof characterized in that the mass of granules have following particle size distribution : granules are retained by separation with different sieves showing retain of 20-28% of granules with a sieve of 0.850 mm, 10-18 % of granules with a sieve of 0.500 mm, 13-20 % of granules with a sieve of 0.180 mm and 17-30 % of granules with a sieve of 0.106 mm .

Most preferably distribution of sodium fusidate granules that are retained by separation with different sieves showing retain of 25 % of granules with a sieve of 0.850 mm, 14 % of granules with a sieve of 0.500 mm, 15 % of granules with a sieve of 0.180 mm and 20 % of granules with a sieve of 0.106 mm.

The advantageous features and preferred embodiments of the present invention summarized in the following items further contribute to solving the object of the invention.

The granules are obtained by a method comprising compaction of a dry mixture and/or granulated and/or coated fusidic acid or a salt thereof with or without excipients performed to obtain granules.

In the present invention, it is provided that a process for the preparation of fusidic acid sodium salt tablets without enteric coating,preferably formed via dry granulate of compacted powdered fusidic acid sodium salt.

One or more times compaction and sieving can be performed. Preferably after first compaction and sieving a second compaction and sieving is performed. Particle size of compacted material reduced of to the proper size by sieving.

The tablet comprising sodium fusidate according to present invention may combine with an excipient, wherein the excipient comprises an additive selected from the group consisting of binder, diluent, lubricant, disintegrant, glidant or mixtures thereof. Further useful additives may include, alone or in combination, buffer agents, stabilizers, fillers, plasticizers, colorants, without being limited thereto.

Examples of binders are, but are not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylpyrrolidone, gelatin, polyethylene glycol, starch, pre-gelatinized starch, sugars, trehalose, glucose, tragacanth, sorbitol, acacia, alginates, carrageenan, xanthan gum, locust bean gum and gum arabic, waxes, polyacrylamide, mixtures thereof, and whatsoever.

Examples of diluents are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst™, mixtures thereof and the like.

Examples of lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like.

Examples of disintegrants are, but not limited to, sodium starch glycolate, sodium carboxymethyl cellulose, calcium carboxymethyl cellulose, croscarmellose sodium, crospovidone, chitosan, agar, alginic acid, calcium alginate, methyl cellulose, microcrystalline cellulose, powdered cellulose, lower alkyl substituted hydroxypropyl cellulose, hydroxylpropyl starch, low-substituted hydroxypropyl cellulose, polacrilin potassium, starch, pregelatinized starch, sodium alginate, magnesium aluminum silicate, polacrilin potassium, povidone, sodium starch glycolate, mixtures thereof and the like.

Examples of glidants are, but not limited to, silicon dioxide, colloidal silicon dioxide, calcium silicate, magnesium silicate, magnesium trisilicate, talc, starch, mixtures thereof or whatsoever.

Examples of buffering agents are, but are not limited to, citric acid, sodium citrate, sodium phosphate, potassium citrate, and mixtures thereof. A preferred buffering agent of the present invention is a mixture of citric acid and sodium citrate.

Examples of stabilizing agents are, but not limited to, polyvinyl pyrrolidone, polymethacrylate, polyvinyl acetate phthalate, alkyl cellulose, hydroxyl alkyl cellulose, polyethylene glycol, polyethylene castor oil, polyethylene glycol sorbitan fatty acid, polyethylene polypropylene glycol, polyethylene oxide, polyoxyethylene alkyl ether, polyoxyethylene stearate, caproic acid, caprilic acid, capric acid, lauric acid, myhstic acid, palmitic acid, stearic acid, arachidic acid, behenic acid, and lignoceric acid, hydrogenated coconut fatty acids, hydrogenated palm fatty acids, hydrogenated rapeseed fatty acids, hydrogenated tallow fatty acids, and hydrogenated castor oil fatty acids, 2-ethylhexanoic acid, isostearic acid, methanol, ethanol, n-propanol, n-butanol, n- pentanol, lauryl alcohol, myristyl alcohol, palmyl alcohol, stearyl alcohol, behenyl alcohol, isopropanol, isobutanol, sec-butanol, 2-ethylhexanol, isododecyl alcohol, isotridecyl alcohol, isostearyl alcohol, alcohol 2-octyldodecyl, glycolic acid, lactic acid, glyceric acid, tartronic acid, malic acid, and citric acid, ethylenglycol, diethylenglycol, triethylenglycol, propylenglycol, dipropylenglycol, glycerin, diglycehn, triglycerin, sorbitol and sorbitan, sodium palmitate, sodium stearate, potassium stearate, potassium palmitate, and sodium myristate, isopropyl myristate, isopropyl palmitate, isopropyl stearate, n- butyl stearate, 2-ethylexyl palmitate, 2-ethylexyl stearate, 2-octyldodecyl myristate, 2-ethylhexyl isostearate, isostearyl isostearate, isononyl isononanoate, thethyl citrate, butyl citrate, methyl lactate, ethyl lactate, n-butyl lactate, and n-propyl lactate, diethylenglycol monoethyl ether, ethylenglycol monobutyl ether, diethylenglycol monobutyl ether, triethylenglycol monobutyl ether, thethylenglycol monoethyl, ethylenglycol monomethyl ether, diethylenglycol mono-n-propyl ether, 2-octyldodecyl myhstate, isostearyl isostearate, thethyl citrate, triethyl citrate, mixtures thereof and the like.

Examples of pharmaceutically acceptable fillers are, but are not limited to, confectioner's sugar, compressible sugar, dextrates, dextrin, dextrose, lactose, mannitol, microcrystalline cellulose, powdered cellulose, sorbitol, sucrose and talc.

Examples of plasticizers are, but are not limited to, water; citrate esters (e.g., triethylcitrate, triacetin); low molecular weight poly(alkylene oxides) (e.g., poly(ethylene glycols), poly(propylene glycols), poly(ethylene/propylene glycols)); glycerol, pentaerythritol, glycerol monoacetate, diacetate or triacetate; propylene glycol; sodium diethyl sulfosuccinate; and the therapeutic compound itself.

Examples of coloring agents are, but are not limited to, red oxide of iron, yellow oxide of iron, and ready color mix system like opadry.

One or more of these additives can be selected and used by the skilled in the art considering the particular desired properties of solid oral dosage form. The amount of all these additives may vary within the ranges conventional in the art.

The compression of the granules into tablet cores can be achieved by any method used in the art such as conventional tabletting machine, eccentring tabletting machine or a rotary compression machine.

The tablets obtained by this invention were further coated by using any of the conventional coating methods known in the art such as pan or perforated pans, spray coating using a fluidized bed granulator, a centrifugal fluidized bed coater or a spray drier or coating with a rotary granulator etc.

These coats comprised of one or more excipients selected from the group comprising film formers or coating agents, opacifiers, taste-masking agents, coloring agents, antitacking agents and the like.

Examples of pharmaceutically acceptable coating agents or film formers are, but not limited to, polysaccharides such as maltodextrin; alkyl cellulose such as methyl or ethyl cellulose; hydroxyalkylcelluloses; polyvinypyrilidone, polyvinyl alcohol, copolymers of vinylpyrrolidone and vinyl acetate and polymers based on methacryclic acid such as Eudragit. These may be applied from aqueous or non-aqueous systems or combinations thereof.

Additives can be included along with the film formers to obtain suitable films. These additives are, but not limited to, plasticizers such as dibutyl phthalate, triethyl citrate, polyethylene glycol polyethylene derivatives such as polysorbate 80 and the like; antitacking agents such as include talk, stearic acid, magnesium stearate, colloidal silicon dioxide and the like.

Opacifying agents are, but not limited to, titanium dioxide, ferric oxide and the like.

When the powder mixture of a tablet formulation does not have the flow characteristics or low bulk density, which is necessary for tablet formation, the Roller compaction method can be used to overcome such problematic. The equipment known as the roller compactor uses pressure to compact, densify and to bind the powders used in tablet formulation into granules.

In this invention, this compaction procedure is preferably performed twice to provide Na fusidate flowability after filler was added. However, performing this compaction method more than two times may be a bit inconvenient, since the art teaches that microcrystalline cellulose shows reduced compactability after repeated compaction events. After the first compaction, porosity of the microcrystalline cellulose is reduced and is associated with reduced compactability of the compacted microcrystalline cellulose relative to the microcrystalline cellulose before the first, or primary, compaction. Thought not being bound by any theory or explanation, it is thought that a number of hydrogen bonds are formed during the primary compaction, reducing the number of potential hydrogen bonding sites for the second or subsequent compaction.

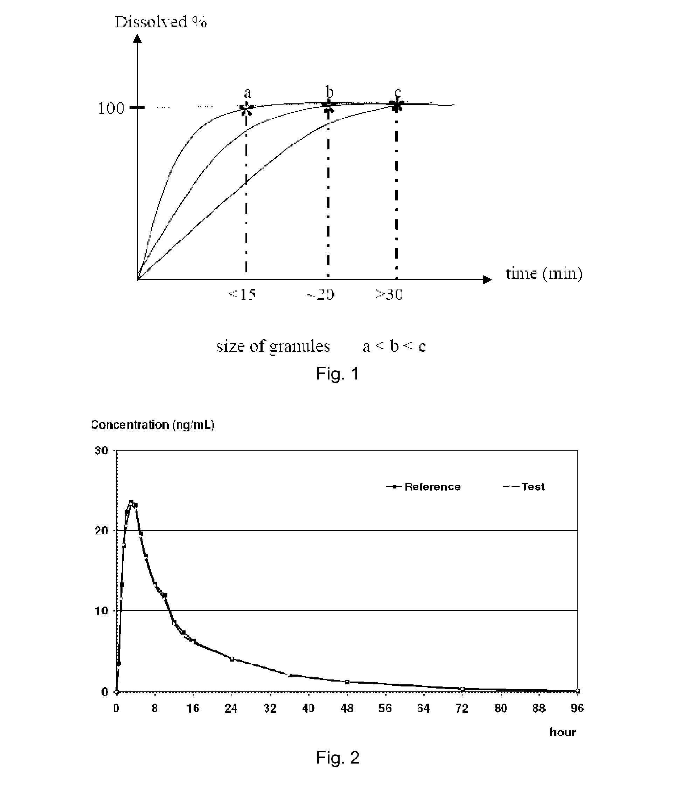

In this invention, the size of Na fusidate granules are important for an appropriate profile of the drug. Because of this reason, the size reducing of granules produced by dried compaction is essential. During a granulation process as the size of granules increases, the number of particles obtained decreases. This step, actually, determines the ratio of excipients to active agent particles. This ratio has a great effect on disintegration of a drug, in other words on dissolution profile of it. Thus, in this invention, to provide a proper dissolution profile some trials were performed to find out the appropriate weight ratio of excipients to active agent particles. The results can be summarized in a graph (Figure 1)

To achieve a more consistent particle size distribution of Na fusidate particles for use in making tablet, granules are passed through a series of separation filters having different openings ranging from 0.850 to 0.106 mm.

The distribution of Na fusidate granules that are retained by each separation screen shown the following percentages;

**Table 8**

| Sieve size (mm) | Preferred (%) | More Preferred (%) | Most Preferred (%) |
|---|---|---|---|
| 0.850 | 10-35 | 20-28 | 25 |
| 0.500 | 0-25 | 10-18 | 14 |
| 0.180 | 5-30 | 13-20 | 15 |
| 0.106 | 10-50 | 17-30 | 20 |

Drug absorption from a solid dosage form after oral administration depends on the release of the drug substance from the drug product, and in vitro dissolution of the test product may be relevant to the prediction of its in vivo performance. As a result, the in vitro specifications for products should be established based on a dissolution profile.

According to this invention dosage form comprising the amount of fusidic acid or salts thereof is higher than 250 mg preferably 500 mg of fusidic acid or salts thereof. More preferably oral dosage form of 500 mg is sodium salt of fusidic acid and oral dosage form of 500 mg fusidic acid sodium is bioequivalent with two 250 mg Fucidin® fusidic acid sodium salt tablets. Dosage form of 500 mg fusidic acid sodium is a tablet or a capsule but tablet is preferred.

In this invention, a comparative dissolution study was carried out in water. The following compositions were tested

**[0054]** - Fucidin ® having 250 mgx2 sodium fusidate as reference product

**[0055]** - Immediate release tablets comprising 500 mg of sodium fusidate as test product comprising sodium fusidate granules having particle size in the interval determined by this invention.

Any suitable dissolution method could be used to reach dissolution profiles. In this invention USP Type-2 apparatus is preferred and used. According to the method which was developed during drug development process, the tablets prepared in accordance with the present invention should exhibit the following dissolution profile when tested in a USP Type 2 apparatus, at 50 rpm, and 37° C and in deionized water medium.

To determine the similarity between the dissolution profiles of the test and reference product a simple model independent approach, that is $f_2$ (similarity factor), was carried out. As per US FDA, $f_2$ values should lie between 50-100 for developing two similar dissolution profiles.

The similarity factor $f_2$ is a measurement of the similarity through a point by point comparison as shown in equation 1.

Equation 1:Similarity Factor

$$f_2 = 50 * \log \frac{100}{\sqrt{1 + \frac{1}{n} \sum_{t=1}^{n} (R_t - T_t)^2}}$$

n: is the number of sampling time points

Rt : is the amount drug released from a reference batch at time t

Tt : is the amount drug released from a test batch at time t.

Generally, f2 values greater than 50 ensure sameness of the performance of the reference product and test product.

The results obtained are summarized below table

Table 1

| Time (min) | % Drug Release; 50 rpm | |
| --- | --- | --- |
| | Two Fucidin® 250 mg Film Tablets (Reference) | 500 mg Film Tablet (Test) |
| 5 | 30,2 | 21,6 |
| 10 | 63,7 | 53,5 |
| 15 | 84,2 | 80,3 |
| 20 | 91,0 | 94,0 |
| 30 | 94,3 | 98,2 |
| 45 | 96,1 | 100,3 |
| 60 | 97, 7 | 101,0 |
| f2 | | 61.0 |

In addition, to prove the effect of Na fusidate granule particle size on dissolution profile, the dissolution test was performed with immediate release tablets comprising 500 mg of sodium fusidate, comprising sodium fusidate granules having a particle size which is out of the interval determined by this invention. The results obtained are summarized below table

Table 2

| Time (min) | % Drug Release; 50 rpm | |
| --- | --- | --- |
| | Two Fucidin® 250 mg Film Tablets (Reference) | 500 mg Film Tablet (Test) |
| 5 | 33,2 | 16,3 |
| 10 | 66,4 | 45,1 |
| 15 | 90,4 | 67,8 |
| 20 | 94,6 | 87,4 |
| 30 | 97,9 | 101,6 |
| 45 | 99,0 | 102,0 |
| 60 | 99,2 | 102,2 |
| f2 | | 42.9 |

The above results clearly show that the $f_2$ values are slightly different. Moreover, while one of the result is acceptable, the other with 42.9 $f_2$ value is not in the limits of 50- 100 as established by the US FDA for claiming similarity between the dissolution profiles of the test and reference product.

According to the bioequivalence study in human volunteers, related to test product and reference product, a 500 mg fusidic acid sodium salt tablet developed in this invention was demonstrated to be bioequivalent against two tablets of Fucidin® 250 mg fusidic acid sodium salt tablets.

Bioequivalence data for the sodium fusidate 500 mg tablets in comparison with two Fucidin® 250 mg film tablets is shown Figure 2 (Sodium Fusidate mean plasma concentrations (all subjects))

The results suggest that these two sodium fusidate tablet formulations (test product) are bioequivalent when orally administered a 500-mg dose of sodium fusidate tablet against two Fucidin® 250 mg film tablets. This result was consistent with the in vitro dissolution of these two formulations.

**Claims**

1. An oral dosage form containing fusidic acid or salts thereof , wherein oral dosage form is prepared by using of granules of fusidic acid or salts thereof **characterized in that** the mass of granules comprising - at least 50% of fusidic acid or a salt thereof granules have diameters greater than 100 $\mu$m and - maximum 50% of fusidic acid or a salt thereof granules have diameters smaller than 100 $\mu$m

2. According to claim 1, granules of fusidic acid or salts thereof **characterized in that** the mass of granules preferably comprising - 70% of fusidic acid or a salt thereof granules have diameters greater than 100 $\mu$m and - 30% of fusidic acid or a salt thereof granules have diameters smaller than 100 $\mu$m

3. An oral dosage form containing fusidic acid or salts thereof wherein said dosage form is prepared from granules wherein the mass of granules of fusidic acid or salts thereof **characterized in that** the mass of granules have following particle size distribution : granules are retained by separation with different sieves showing retain of 10-35% of granules with a sieve of 0.850 mm, 0-25 % of granules with a sieve of 0.500 mm, 5-30 % of granules with a sieve of 0.180 mm and 10-50 % of granules with a sieve of 0.106 mm

4. According to claim 3, the mass of granules of fusidic acid or salts thereof **characterized in that** the mass of granules have more preferably following particle size distribution : granules are retained by separation with different sieves showing retain of 20-28% of granules with a sieve of 0.850 mm, 10-18 % of granules with a sieve of 0.500 mm, 13-20 % of granules with a sieve of 0.180 mm and 17-30 % of granules with a sieve of 0.106 mm

5. According to claim 4, the mass of granules have most preferably following particle size distribution : granules are retained by separation with different sieves showing retain of 25% of granules with a sieve of 0.850 mm, 14 % of granules with a sieve of 0.500 mm, 15 % of granules with a sieve of 0.180 mm and 20 % of granules with a sieve of 0.106 mm

6. The granules according to preceding claims are obtained by a method comprising compaction of a dry mixture and/or granulated and/or coated fusidic acid or a salt thereof with or without excipients performed to obtain granules

7. According to claim 6 said method comprising one or more times compaction and sieving are performed

8. According to claim 7 said method comprising after first compaction and sieving a second compaction and sieving is performed

9. According to claim 8 said method includes reducing of particle size of compacted material to the proper size by sieving.

10. According to claims of 1 to 4 oral dosage form comprising the amount of fusidic acid or salts thereof is higher than 250 mg

11. According to claim 10 oral dosage form comprises 500 mg of fusidic acid or salts thereof

12. According to claim 11, oral dosage form of 500 mg is sodium salt of fusidic acid and oral dosage form of 500 mg fusidic acid sodium is bioequivalent with two 250 mg Fucidin® fusidic acid sodium salt tablets.

13. According to claim 12, oral dosage form of 500 mg fusidic acid sodium has a similarity factor of over 50 in vitro dissolution environments when compared with two 250 mg Fucidin® fusidic acid sodium salt tablets.

14. According to claim 13 oral dosage form of 500 mg fusidic acid sodium is a tablet or a capsule.

Fig. 1

Fig. 2

Europäisches
Patentamt

European
Patent Office

Office européen
des brevets

# EUROPEAN SEARCH REPORT

Application Number

EP 10 15 7866

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 96/03128 A1 (LEO PHARM PROD LTD [DK]; RASMUSSEN ERIK PREBEN [DK]) 8 February 1996 (1996-02-08) | 1,2,6-9 | INV.<br>A61K9/16<br>A61K31/575 |
| Y | * page 2, line 14 - page 3, line 28 *<br>* examples 1,2 *<br>* claims * | 3-5,<br>10-14 | |
| X | US 4 191 743 A (KLEMM KLAUS [DE] ET AL) 4 March 1980 (1980-03-04) * column 1, line 56 - column 2, line 39 * * column 3, line 4 - line 26 * * claims * | 1,2,6 | |
| Y | WO 89/00424 A1 (LEO PHARM PROD LTD [NL]) 26 January 1989 (1989-01-26) * examples 1-3, * | 1-14 | |
| Y | US 3 205 135 A (LEIF TYBRING) 7 September 1965 (1965-09-07) * examples 1,3,4 * | 1-14 | |
| | | | TECHNICAL FIELDS SEARCHED (IPC) |
| | | | A61K |

The present search report has been drawn up for all claims

| Place of search | Date of completion of the search | Examiner |
|---|---|---|
| Munich | 5 October 2010 | Giró, Annalisa |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 10 15 7866

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2010

| Patent document cited in search report | | Publication date | Patent family member(s) | | Publication date |
|---|---|---|---|---|---|
| WO 9603128 | A1 | 08-02-1996 | AT | 209496 T | 15-12-2001 |
| | | | AU | 681980 B2 | 11-09-1997 |
| | | | AU | 3164495 A | 22-02-1996 |
| | | | CA | 2193235 A1 | 08-02-1996 |
| | | | CN | 1154066 A | 09-07-1997 |
| | | | CZ | 9700229 A3 | 16-04-1997 |
| | | | DE | 69524226 D1 | 10-01-2002 |
| | | | DE | 69524226 T2 | 27-06-2002 |
| | | | DK | 773783 T3 | 02-04-2002 |
| | | | EP | 0773783 A1 | 21-05-1997 |
| | | | ES | 2163521 T3 | 01-02-2002 |
| | | | FI | 965214 A | 27-12-1996 |
| | | | HU | 76836 A2 | 28-11-1997 |
| | | | JP | 10502937 T | 17-03-1998 |
| | | | JP | 3853358 B2 | 06-12-2006 |
| | | | NZ | 290806 A | 26-01-1998 |
| | | | PL | 318261 A1 | 26-05-1997 |
| | | | PT | 773783 E | 29-04-2002 |
| | | | RO | 118633 B1 | 29-08-2003 |
| | | | RU | 2140272 C1 | 27-10-1999 |
| US 4191743 | A | 04-03-1980 | DE | 2650306 A1 | 03-05-1978 |
| | | | FR | 2368962 A1 | 26-05-1978 |
| | | | GB | 1534454 A | 06-12-1978 |
| | | | JP | 53061188 A | 01-06-1978 |
| WO 8900424 | A1 | 26-01-1989 | DK | 17590 A | 22-01-1990 |
| | | | EP | 0391898 A1 | 17-10-1990 |
| | | | JP | 3501967 T | 09-05-1991 |
| US 3205135 | A | 07-09-1965 | BE | 619713 A1 | 05-11-1962 |
| | | | GB | 955627 A | 15-04-1964 |

EPO FORM P0459

For more details about this annex : see Official Journal of the European Patent Office, No. 12/82

**EP 2 382 968 A1**

**REFERENCES CITED IN THE DESCRIPTION**

**Patent documents cited in the description**

- WO 9603128 A